# EUROPEAN PATENT SPECIFICATION

(11) **EP 2 027 825 B1**
(45) Date of publication and mention of the grant of the patent: **30.09.2015**
(21) Application number: 08252679.9
(22) Date of filing: 13.08.2008
(51) Int. Cl.: A61B 18/14, A61B 18/00, A61B 18/12

(54) **Electrosurgical system**
Elektrochirurgisches System
Système électrochirurgical

(30) Priority: 24.08.2007 GB 0716590
(43) Date of publication of application: 25.02.2009
(73) Proprietor: Gyrus Medical Limited, Cardiff CF3 0LT (GB)
(72) Inventor: Newton, Michael David, Bassaleg Newport NP10 8JE (GB); Curtis, Richard J., Rogerstone Newport NP10 9GJ (GB); Ford, Andrew J., Easton in Gordano N. Somerset BS20 0QB (GB)
(74) Representative: Wallin, Nicholas James

(56) References cited:
- US-A- 5 425 375
- US-A- 5 487 386
- US-A1- 2006 079 880
- US-B1- 6 651 669

## Description

This invention relates to an electrosurgical system including an electrosurgical generator and electrosurgical instruments for use therewith.

Electrosurgical instruments are frequently recommended as "single use" instruments, meaning that they should be disposed of after use. However, such instruments are sometimes re-used against the wishes of the manufacturers, mostly after re-sterilisation. In fact, "Reprocessing" companies have been set up to take surgical instruments and reprocess them ready for re-use, despite the recommendations of the manufacturers that this is not desirable.

Some systems have been designed to identify when a surgical instrument is being re-used or over-used, and to prevent the activation of such instruments when such activity has been detected. Examples of such systems are to be found in US patents 5,383,874, 5,425,375, 5,487,386, 6,651,669, 5,651,780, 5,400,267, 6,237,604 and others. Frequently, such systems provide a memory device with a unique code within each instrument, and log the usage of such devices by reading the code when the device is connected to a controller or generator.

The present invention attempts to provide an alternative to such re-use detection systems, which allows for a flexible approach capable of allowing, monitoring or preventing re-use, as appropriate.

Accordingly, an electrosurgical system is provided comprising:
a generator for generating radio frequency power, and
an electrosurgical instrument including an electrode assembly, the electrosurgical instrument being detachably connectible to the generator such that radio frequency power can be conveyed to the electrode assembly,
characterised in that the generator includes a counter that is incremented in value by one each time an electrosurgical instrument is detachably connected to the generator, the electrosurgical instrument includes a memory device capable of recording the value of the counter when the electrosurgical instrument is connected to the generator, and the system includes a comparison unit capable of comparing the current value of the counter with the value of the counter stored in the memory device, and generating a signal when the difference between the two values of the counter is greater than a predetermined threshold.

The invention relies on the likelihood that, for a single-use instrument to be reprocessed for re-use, it will need to be sent away for sterilization. While the instrument is being reprocessed, the generator will likely be used with other instruments. Thus, if the incremental counter is incremented every time that an instrument is connected to the generator, the counter will be at a much higher value by the time that a reprocessed instrument is re-presented to the generator. This difference between the original value of the counter and the current value of the counter can be used to indicate the likelihood that there is an attempt to re-use a previously-used instrument.

Preferably, the predetermined threshold between the two values of the counter is greater than one. In this way, should there be a problem with the equipment before or during a surgical procedure, which necessitates the disconnection and reconnection of the surgical instrument from the generator, the continued use of the instrument is allowed. This means that the re-use detection system does not operate in legitimate circumstances where reconnection of an electrosurgical instrument is essential, for example to continue with a procedure that is somehow interrupted or continued after a break. On the other hand, the system will serve to identify situations where the gap between the original use and the attempted re-use is so large that reprocessing of a single use instrument is the most likely cause. Conveniently, the predetermined threshold is between 5 and 10, allowing this many reconnections (or the use of other instruments in between) before a re-use signal is generated.

In some circumstances the predetermined threshold is greater than 10. This would be appropriate for generators where the usage is relatively heavy, and the counter would most likely be well advanced by the time that any reprocessed instruments were returned to the operating theater. The threshold may be set differently for generators in different hospitals or surgical practices, or for different countries or regions. Additionally or alternatively, the threshold may be set differently for one type of instrument as opposed to another. For example, an instrument used for a first procedure may need to be disconnected and reconnected during the procedure, whereas an instrument used for a different procedure may not. Different thresholds can therefore take into account these differences in the accepted usage of these instruments.

The comparison unit is conveniently present in the generator. In this arrangement, the generator reads the stored counter value from the memory of the electrosurgical instrument, and compares it with the current value of the counter within the generator. If the difference is above the threshold, a signal is generated to indicate an attempt to re-use the instrument. It is possible that the generator may have a plurality of incremental counters, one for each type of instrument used therewith. Most electrosurgical systems have some form of identification system for detecting which type of instrument is connected to the generator. In this arrangement, the generator increments the counter appropriate for whichever type of instrument is detected, and so an independent record of the usage of different instruments is obtained. Additionally or alternatively, a single incremental counter can be incremented whenever an instrument is connected to the generator, regardless of which type of instrument is connected.

Conceivably, the comparison unit is present in the electrosurgical instrument. In this arrangement, the generator writes the value of the counter to a first memory within the instrument when the instrument is first connected to the generator. When the instrument is reconnected, the generator writes the current value of the counter to a second memory within the instrument. The comparison circuitry within the instrument compares the values in the first and second memories, and establishes whether the difference therebetween is greater than the allowed threshold. If the difference is too great, a re-use signal is generated and sent to the generator.

According to one arrangement, the generator displays a warning message in response to the signal. This alerts the user to the suspicion that the instrument presented to the generator may have been reprocessed. The generator may still allow the reprocessed instrument to be used, or may alternatively prevent the usage thereof. Additionally or alternatively, the generator generates an audible alarm in response to the signal. Once again, this will alert the user to the use of a reprocessed instrument. Preferably, the generator blocks the supply of radio frequency power to the electrode assembly in response to the signal. Alternatively, the generator may allow the reprocessed instrument to be used, but at a reduced power setting in order to offset any impaired performance or reduced safety margins that may have been introduced by the reprocessing of the instrument.

Various combinations of the above may conveniently be employed. For example, the generator may create a log of the re-use of the instruments, capable of being downloaded by a service engineer to keep track of the usage of the generator. Additionally or alternatively, the generator or the memory device stores information regarding the number of times the signal has been generated when the electrosurgical instrument has been connected to the generator. Conceivably, the generator makes a different response depending on the number of times that the signal has been generated. Thus, if the difference between the counter values indicated that a single use instrument was being re-used for the first time, re-use may be permitted or permitted at a lower power setting. If the same instrument was subsequently detected as being presented to the generator for use a third or subsequent time, the activation of the instrument could be prevented, or allowed only at progressively lower and lower power settings in order to preserve the appropriate safety margins. Additionally or alternatively, the visual or audible indications can change in content or severity as further re-use is detected, thereby warning the user that the risk of impaired performance is increasing.

The invention further resides in a method of monitoring re-use of an electrosurgical instrument comprising the steps of
i) detachably connecting the electrosurgical instrument to an electrosurgical generator,
ii) detecting whether the electrosurgical instrument has been detachably connected previously to an electrosurgical generator,
iii) if the electrosurgical instrument has not been detachably connected previously to an electrosurgical generator, writing a value from an incremental counter housed in the generator to a memory present in the electrosurgical instrument, and
iv) incrementing the incremental counter in value by one in response to the detachable connection of the electrosurgical instrument.

The method preferably also includes the additional steps of
v) if the electrosurgical instrument has been connected previously to an electrosurgical generator, comparing the present value of the counter with the value stored in the memory present in the electrosurgical instrument, and
vi) generating a signal if the difference between the current value and the stored value exceeds a predetermined threshold.

The invention will now be described in more detail, by way of example only, with reference to the accompanying drawings, in which;
Figure 1 is a diagrammatic representation of an electrosurgical system in accordance with the invention;
Figure 2 is a circuit diagram of an electrosurgical instrument and identification circuitry for allowing an electrosurgical generator to be adjusted in response to connection of an electrosurgical instrument including a passive electrical identification component;
Figure 3 is a set of three waveform diagrams relating to the circuitry of Figure 2;
Figure 4 is a circuit diagram of a connector forming part of an electrosurgical instrument which itself forms part of the electrosurgical system of Figure 1;
Figures 5 & 6 are flow charts showing the operation of the invention in accordance with one embodiment thereof, and
Figure 7 is a flow chart showing the operation of the invention in accordance with an alternative embodiment thereof.

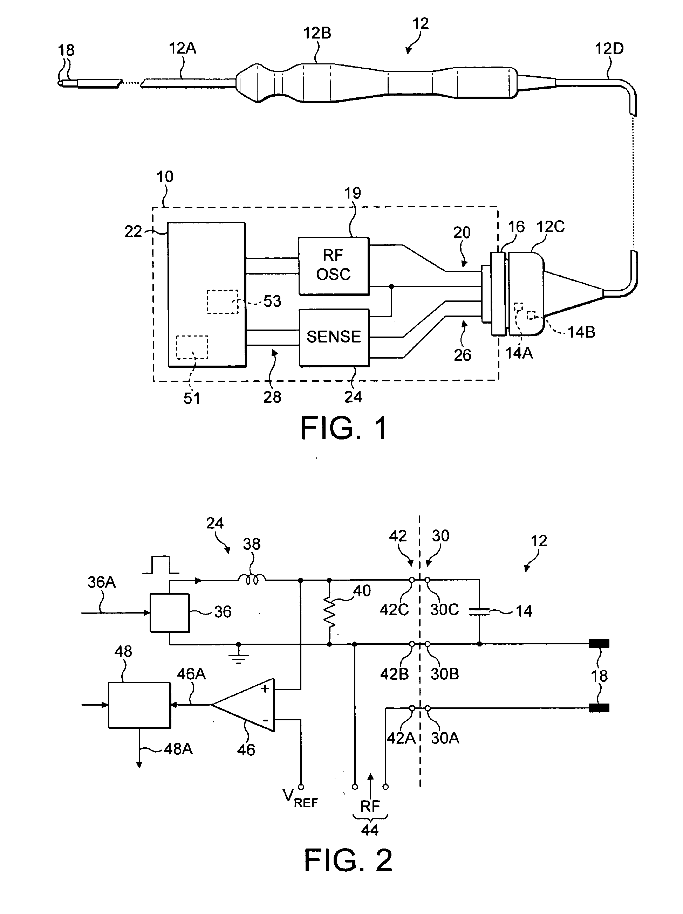

Referring to Figure 1, an electrosurgical system in accordance with the invention comprises a first unit in the form of an electrosurgical generator 10 for generating radio frequency power, and a second unit in the form of an electrosurgical instrument 12 in the form of a pencil-grip handpiece and including an electrode assembly, a handpiece body 12B, and a connector 12C which is coupled to the handpiece body and the electrode assembly by a cable 12D. Housed within the connector 12C are two passive electrical identification components 14A, 14B which, in this case, are capacitors. The connector 12C is a multiple contact plug which mates with a multiple contact socket 16 on the generator 10 so that the instrument 12 can be removably connected to the generator.

In this embodiment of the invention, the electrode assembly 12A is a bipolar assembly having two tissue treatment electrodes 18. However, the assembly may comprise single or multiple electrode elements, in other words, monopolar, tripolar or multiple electrode assemblies. In the illustrated embodiment, the electrodes 18 are connected via respective electrical conductors running through the handpiece body 12B and the cable 12D to a respective pair of contacts (not shown in Figure 1) in the plug 12C. The capacitor 14 is also connected to a respective pair of contacts (not shown in Figure 1) in the plug 12C.

The generator comprises a radio frequency (RF) oscillator 19 with a pair of RF output lines 20 for feeding RF energy via the socket 16 to the RF power contacts in the instrument plug 12C for energising the bipolar electrodes 18. The RF oscillator 19 is controlled by a controller 22 which has connections to a user interface (not shown). Coupled to the controller is an electrode identification circuit 24 having connections 26 to the socket 16 for connecting to the capacitors 14A, 14B. When the instrument 12 is coupled to the generator 10, the electrode identification circuit 24 can be used to measure the value of the capacitors, detector output signals being conveyed by lines 28 to the controller 22 for controlling the RF oscillator 19 in response to the value of the capacitors 14A, 14B. It will be appreciated that, by providing capacitors 14 of different values in different instruments 12, the value of the capacitors can be used to identify the instrument 12 and thereby cause adjustment of the generator RF output to suit each respective instrument when it is connected. To this extent, the system operates largely as described in U.S. Patent No. 6,074,386, the entire disclosure of which is incorporated in the present specification by reference.

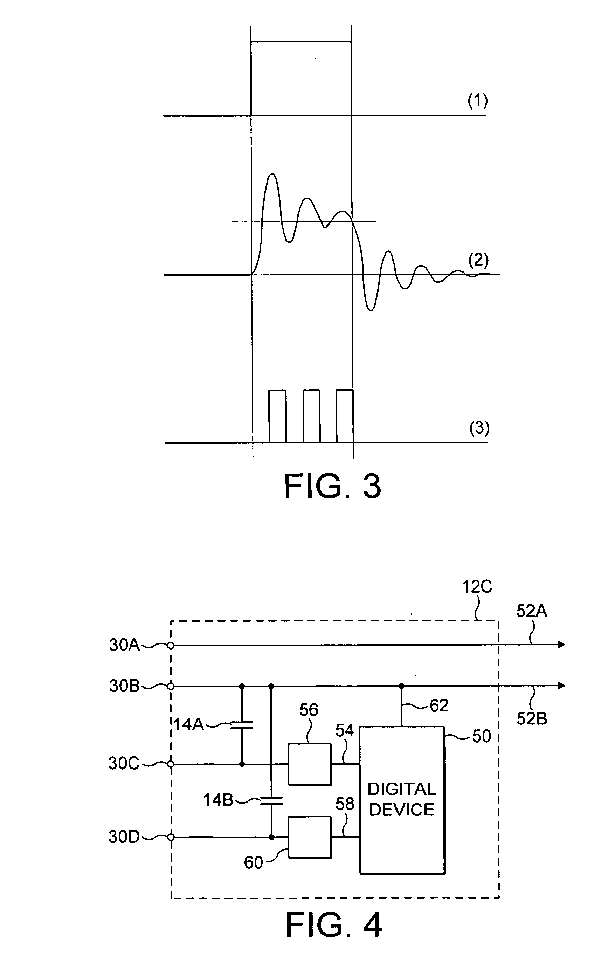

Measurement of capacitor values in the system will now be described with reference to the simplified circuit diagram of Figure 2 and the accompanying waveform diagrams of Figure 3.

Referring to Figure 2, the electrosurgical instrument 12 (in the form of an electrode assembly having two electrodes 18) has a set of contacts 30, two of which constitute a pair of RF power contacts (30A, 30B) which are coupled to the electrodes 18 of the instrument. An identification capacitor 14 is connected between one of the RF power contacts 30B and a third contact 30C of the set so that the electrodes 18 and the capacitor 14 have one common contact 30B. On the generator side, the electrode identification circuit comprises a signal source 36 having a timing input 36A connected to the controller. The source 36 is configured to generate an interrogation pulse across a pair of source output lines 36B. Connected in series between the source 36 and the capacitor 14 is an electrical component in the form of an inductance 38. Downstream of the inductor 38 is a shunt-connected damping resistance 40.

The generator has a set of contacts 42 which mate with the contacts 30 of the instrument 12, as shown. Output lines 44 from the RF oscillator 19 (not shown in Figure 2) are coupled to contacts 42A, 42B which mate with contacts 30A, 30B of the instrument 12 so that electrosurgical RF energy is conveyed to the electrodes 18 when the instrument 12 is connected to the generator. A third contact 42C on the generator is connected via the inductor 38 to one of the output lines 36B of the signal source 36, whilst the contact 42B which mates with instrument contact 30B acting as a common contact for the capacitor 14 and one of the electrodes 18 is not only connected to the output lines of the RF oscillator, but also to the other output line 36B of the signal source 36.

It will be appreciated that when the instrument 12 is connected to the generator, the capacitor 14 in the instrument and the series inductance 38 in the identification circuit 24 together form a series-resonant combination circuit having a resonant frequency determined by the values of the capacitor 14 and the inductor 38. Since capacitor 14 has different values depending uniquely on the instrument 12 in which it is contained, the resonant frequency identifies the instrument 12.

Coupled to the connection between the inductor 38 and the capacitor 14 is one input of a comparator 46 the other input of which is connected to a reference voltage source VREF. This reference voltage is at a predetermined potential with respect to the other arm of the resonant circuit formed by capacitor 14 and inductor 38 (here the output line 36B of the signal source which is not connected to the inductor 38). Comparator 46 has an output connected to a signal processing circuit 48 which, in turn, feeds the controller 22 (see Figure 1) via its output 48A.

It will be appreciated that when the voltage step-change represented by the leading edge of the interrogation pulse generated by signal source 36 is applied to the resonant combination of capacitor 14 and inductor 38, a ringing signal is generated at the junction between capacitor 14 and inductor 38, the ringing occurring at the resonant frequency referred to above. Owing to the presence of the parallel resistance 40, the ringing signal decays predictably. In practice, the value of the resistance 40 is chosen such that its effect upon the decay rate of the ringing of the signal is minimised, but its effect in the presence of noise is maximised, its main purpose is for EMC protection and to keep the ringing of the resonant network to predictable values. The value of the voltage reference source VREF is selected such that, during the interrogation pulse, the ringing signal crosses over the reference voltage several times, with the effect that a corresponding binary signal appears at the output 46A of the comparator 46, the binary signal taking the form of a squarewave having a repetition rate equal to the resonant frequency of the capacitor/inductor combination. In this example, the signal processing circuit 48 measures the interval between successive edges of the squarewave, thereby detecting the pulse width of the squarewave signal and, hence, the period of the ringing signal. As an alternative, signal processing circuit 48 may employ a counter arranged to count the number of changes of state of the output signal from the comparator 46 as a means of determining the ringing frequency or period.

The interrogation pulse is shown by waveform diagram (1) in Figure 3. The ringing signal is shown in diagram (2) and the squarewave signal outputted by the comparator 46 is shown by diagram (3) in Figure 3.

It will be appreciated that detecting the transient response of the resonant combination of the capacitor 14 and inductor 38 rather than using the resonant combination to determine the frequency of oscillation of an identification circuit oscillator as in U.S. 6,074,386, allows the contacts coupling the identification capacitor 14 to the identification circuit of the generator 10 to be used for different purposes at times other than during the transient response. In other words, sensing oscillations in the resonant combination only for a short period permits sharing of the connections, in ways that will be described below.

In the system described above with reference to Figure 2, the identification components (capacitors 14A, 14B) are housed a connector 12C forming part of an instrument 12 detachably connected to the generator 10. In this case, the generator 10 constitutes the "first unit" and the complete instrument 12 constitutes the "second unit", the connection interface occurring between the plug 12C and the socket 16. In an alternative embodiment, the first unit may be an instrument body and the second unit a sterilisable instrument part (not shown). Thus, the instrument 12 may have a detachable electrode assembly 12A and the capacitor may be housed in the detachable part, so that the connection interface, for the purpose of identification, is not between the plug 12C and the socket 16 but between mutually separable parts of the instrument 12.

Referring to Figure 4, in a preferred embodiment of the invention, the connector 12C houses two identification components 14A , 14B (e.g. two capacitors of different value. The case of the two values of capacitance being equal is used as an error or fault condition indicator, which may be as a result of a short circuit in the plug, etc. The connector 12C also houses a digital device 50 which operates through the same contacts of the connector 12C as the capacitors 14A, 14B. In this case, the connector 12C has a contact set 30 comprising four contacts two of which 30A, 30B are used for RF power and are coupled to lines 52A, 52B passing through the connector to the cable 12D and electrodes 18 (see Figure 1). In this case, both capacitors 14A, 14B have one terminal connected to one of the RF power contacts 30B. The other terminals are connected to respective identification contacts 30C, 30D. However, these identification contacts 30C, 30D are also used for functions associated with the digital device 50. As will be seen from Figure 4, digital device 50 has a power supply line 54 coupled to identification contact 30C via an intermediate circuit 56, and a data output 58 coupled to identification contact 30D via a second intermediate circuit 60. A local 0V signal and power return terminal 62 of the digital device 50 is coupled to the line 52B, thereby sharing contact 30B not only with the electrodes of the instrument, but also with the first capacitor 14A.

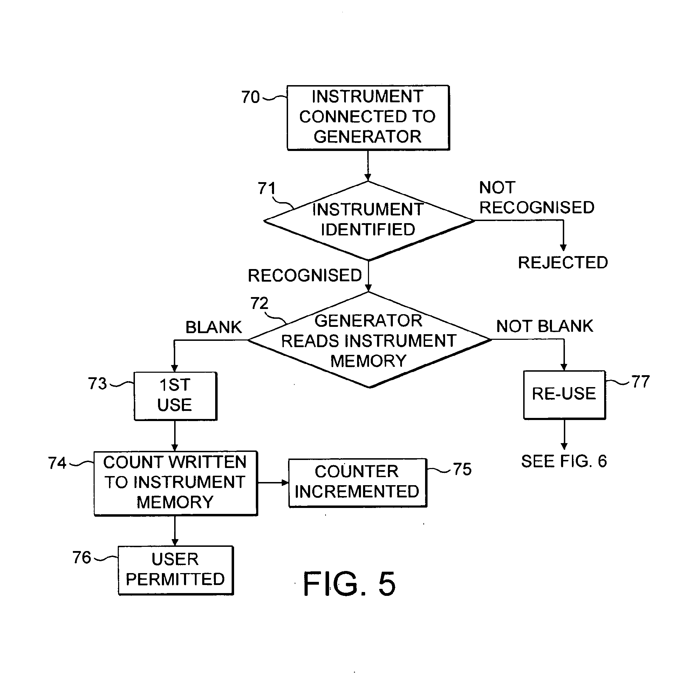

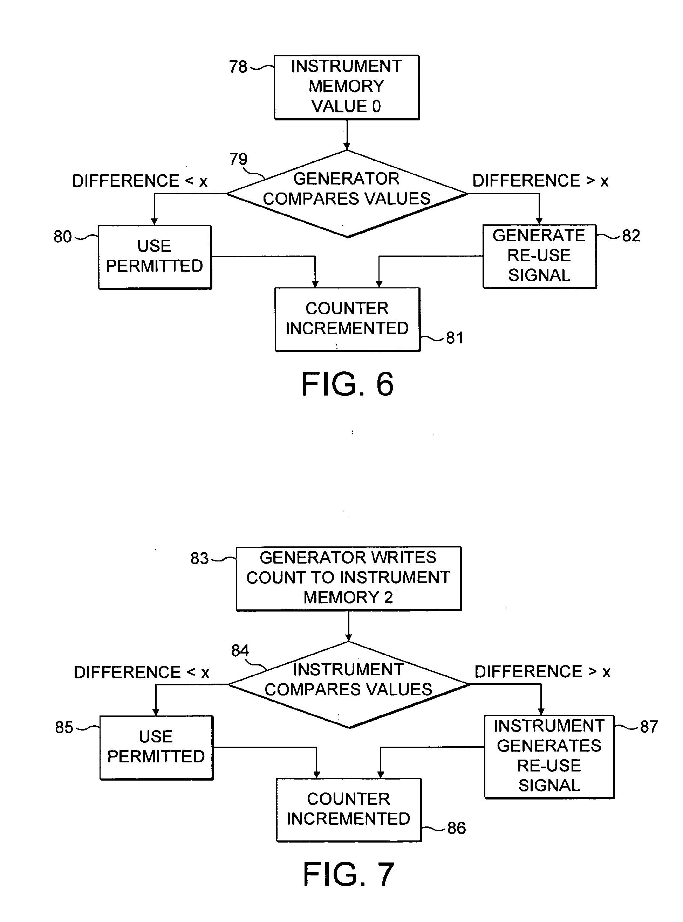

Referring back to Figure 1, controller 22 includes a counter 51 and a comparison circuit 53. The controller 22 and the interconnections between the controller and the digital device 50 in the instrument 12 are configured and arranged such that a count value outputted by the counter 51 can be written to a memory in the digital device 50 and such that values stored in the digital device memory can be read out by the controller. The operation of the re-use detection system will now be described with reference to Figures 1 to 4, and also to the flow diagrams of Figures 5 & 6.

In use, the instrument 12 is connected to the generator 10 (step 70), and the instrument identification is carried out as described above (step 71). If the instrument is not recognized, it is rejected, but if the instrument is recognized as an acceptable instrument the process moves to step 72. In this step, the generator 10 interrogates the memory of the digital device 50 to see if the memory contains a previous value from the counter 51. If the memory is blank, the generator concludes that this is the first use of the instrument 12 (step 73), and writes the current value of the counter 51 (value1) to the digital device (step 74). The counter 51 is then incremented in value by one, to designate that an instrument has been connected to the generator (step 75), and the instrument is authorized for use (step 76).

If the memory is not blank in step 72, then the generator concludes that the instrument 12 has been previously used (step 77), and goes through the steps illustrated in Figure 6. In step 78, the generator reads the value (value0) stored in the memory of the digital device 50 from its previous connection to the generator 10. In step 79, the comparison circuit 53 of the generator compares the stored value (value0) with the current value of the counter (value1). If the difference between the two values is less than a predetermined threshold x (say, for example 10), then the generator permits the use of the instrument 12 (step 80), and increments the counter 51 (step 81). If the difference is greater then the threshold x, then the generator concludes that the instrument 12 is a reprocessed instrument and generates a re-use signal (step 82). Even if the re-use signal is generated, the generator increments the counter 51 to record that a new attempt has been made to connect an instrument to the generator 10.

The controller 22 may be programmed such that when it receives a re-use signal, the activation of the generator with the instrument 12 is prevented, and a display on the generator displays a warning message (such as "Re-used Instrument"). The controller 22 may alternatively be programmed to allow the instrument 12 to be used, but to record the use in a log that can be accessed subsequently. Other options include the sounding of an audible alarm, or the altering of the settings of the generator to allow the operation of the instrument 12 but only at reduced power settings.

Thus, where an original instrument is connected and disconnected to the generator within a reasonably short period of time (during which few if any instruments will have been connected), the difference between the two values (value1 and value0) will be less than the threshold x, and the use of the instrument will be permitted. This ensures that legitimate reasons for disconnecting and reconnecting the instrument do not result in the use of the instrument being barred. However, when the period of time is longer such that the number of uses between the original connection and reconnection means that the difference between the two values (value1 and value0) is greater than the threshold x, the generator concludes that the instrument is a reprocessed instrument and generates the re-use signal at step 82.

Clearly, there may be other similar generators present in the same hospital or surgical center, or in other locations in general. Thus it is possible that the instrument 12 could have been used with one generator in a first location, reprocessed, and presented to a different generator in a different location. In these circumstances, due to the different rates of usage experienced with different generators, it is highly unlikely that the value of the counters of the two different generators will be within the threshold x. Thus the attempted re-use of an instrument will be identified, even if the use is attempted with different generators.

Figure 7 illustrates the process with an alternative version of the re-use identification system, in which the comparison is carried out not within the generator but within the instrument 12. In this arrangement the digital memory device has at least two memory locations (memory1 and memory2), and the comparison circuit 53 is also included within the instrument 12. The first part of the process is similar to that previously described with reference to Figure 5, with the generator 10 reading the memory in the digital device (in this case memory1) to see if it is blank. If it is indeed blank, the current value of the counter (value1) is written to memory1 of the digital device 50 (in step 74).

If memory1 in the digital device 50 is not blank, the generator writes the current value of the counter (value1) to memory2 of the digital device (see step 83 in Figure 7). The comparison circuit 53 within the instrument 12 then compares the values in memory1 and memory2 to see if the difference is greater than the threshold x (step 84). As before, if the difference is less than the threshold x, use is permitted (step 85) and the counter within the generator is incremented (step 86). If the difference is greater than the threshold x, the re-use signal is generated (step 87) and sent to the generator for action.

The invention has been described in such a way that variations to the way the re-use signal is generated, as well as the way in which actions are taken in response to the re-use signal, will be apparent to those skilled in the art. Thus the system may be customised to various circumstances, including the type of instrument connected to the generator, the procedure to be performed, or the country or region of operation.

## Claims

1. An electrosurgical system comprising:
a generator (10) for generating radio frequency power, and
an electrosurgical instrument (12) including an electrode assembly, the electrosurgical instrument being detachably connectible to the generator such that radio frequency power can be conveyed to the electrode assembly,
**characterised in that** the generator includes a counter (51) that is adapted to be incremented in value by one each time an electrosurgical instrument is detachably connected to the generator, the electrosurgical instrument includes a memory device (50) adapted to record the value of the counter when the electrosurgical instrument is connected to the generator, and the system includes a comparison unit (53) adapted to compare the current value of the counter with the value of the counter stored in the memory device, and adapted to generate a signal when the difference between the two values of the counter is greater than a predetermined threshold.

2. An electrosurgical system according to claim 1, **characterised in that** the predetermined threshold is greater than one.

3. An electrosurgical system according to claim 2, **characterised in that** the predetermined threshold is between 5 and 10.

4. An electrosurgical system according to claim 2, **characterised in that** the predetermined threshold is greater than 10.

5. An electrosurgical system according to any preceding claim, **characterised in that** the comparison unit (53) is present in the generator (10).

6. An electrosurgical system according to any of claims 1 to 4, **characterised in that** the comparison unit (53) is present in the electrosurgical instrument (12).

7. An electrosurgical system according to any preceding claim, **characterised in that** the generator (10) is adapted to display a warning message in response to the signal.

8. An electrosurgical system according to any preceding claim, **characterised in that** the generator (10) is adpated to generate an audible alarm in response to the signal.

9. An electrosurgical system according to any preceding claim, **characterised in that** the generator (10) is adpated to reduce the power of the radio frequency supplied to the electrode assembly in response to the signal.

10. An electrosurgical system according to any preceding claim, **characterised in that** the generator (10) is adpated to block the supply of radio frequency power to the electrode assembly in response to the signal.

11. An electrosurgical system according to any preceding claim, **characterised in that** the generator (10) or the memory device (50) is adpated to store information regarding the number of times the signal has been generated when the electrosurgical instrument (12) has been connected to the generator.

12. An electrosurgical system according to claim 11, **characterised in that** the generator (10) is adpated to make a different response depending on the number of times that the signal has been generated.

13. A method of monitoring re-use of an electrosurgical instrument (12) comprising the steps of:
i) detachably connecting the electrosurgical instrument (12) to an electrosurgical generator (10),
ii) detecting whether the electrosurgical instrument has been detachably connected previously to an electrosurgical generator,
iii) if the electrosurgical instrument has not been detachably connected previously to an electrosurgical generator, writing a value from an incremental counter (51) housed in the generator to a memory (50) present in the electrosurgical instrument, and
iv) incrementing the incremental counter in value by one in response to the detachable connection of the electrosurgical instrument.

14. A method of monitoring re-use according to claim 13, **characterised by** the further steps of:
v) if the electrosurgical instrument (12) has been connected previously to an electrosurgical generator (10), comparing the present value of the incremental counter (51) with the value stored in the memory (50) present in the electrosurgical instrument (12), and
vi) generating a signal if the difference between the current value and the stored value exceeds a predetermined threshold.

## Patentansprüche

1. Elektrochirurgisches System, umfassend:
einen Generator (10) zum Generieren von Hochfrequenzenergie und
ein elektrochirurgisches Instrument (12) mit einer Elektrodenanordnung, wobei das elektrochirurgische Instrument mit dem Generator lösbar verbunden werden kann, derart, dass die Hochfrequenzenergie zur Elektrodenanordnung übertragbar ist, **dadurch gekennzeichnet,**
**dass** der Generator einen Zähler (51) umfasst, der derart ausgebildet ist, dass sein Wert bei jedem lösbaren Verbinden des elektrochirurgischen Instruments mit dem Generator um eins erhöht wird,
**dass** das elektrochirurgische Instrument eine Speichereinrichtung (50) umfasst,
die ausgebildet ist für die Speicherung des Werts des Zählers, wenn das elektrochirurgische Instrument mit dem Generator verbunden wird, und
**dass** das System eine Vergleichseinheit (53) umfasst, die ausgebildet ist zum Vergleichen des aktuellen Werts des Zählers mit dem in der Speichereinrichtung gespeicherten Wert des Zählers und zum Generieren eines Signals, wenn die Differenz zwischen den beiden Werten des Zählers größer ist als ein vorgegebener Schwellwert.

2. Elektrochirurgisches System nach Anspruch 1, **dadurch gekennzeichnet, dass** der Schwellwert größer als eins ist.

3. Elektrochirurgisches System nach Anspruch 2, **dadurch gekennzeichnet, dass** der vorgegebene Schwellwert zwischen 5 und 10 liegt.

4. Elektrochirurgisches System nach Anspruch 2, **dadurch gekennzeichnet, dass** der vorgegebene Schwellwert größer als 10 ist.

5. Elektrochirurgisches System nach einem der vorhergehenden Ansprüche, **dadurch gekennzeichnet, dass** die Vergleichseinheit (53) in dem Generator (10) angeordnet ist.

6. Elektrochirurgisches System nach einem der Ansprüche 1 bis 4, **dadurch gekennzeichnet, dass** die Vergleichseinheit (53) in dem elektrochirurgischen Instrument (12) angeordnet ist.

7. Elektrochirurgisches System nach einem der vorhergehenden Ansprüche, **dadurch gekennzeichnet, dass** der Generator (10) ausgebildet ist zur Anzeige einer Warnmeldung in Reaktion auf das Signal.

8. Elektrochirurgisches System nach einem der vorhergehenden Ansprüche, **dadurch gekennzeichnet, dass** der Generator (10) ausgebildet ist zum Erzeugen eines akustischen Alarms in Reaktion auf das Signal.

9. Elektrochirurgisches System nach einem der vorhergehenden Ansprüche, **dadurch gekennzeichnet, dass** der Generator (10) ausgebildet ist zum Reduzieren der Hochfrequenzleistung, die der Elektrodenanordnung zugeführt wird, in Reaktion auf das Signal.

10. Elektrochirurgisches System nach einem der vorhergehenden Ansprüche, **dadurch gekennzeichnet, dass** der Generator (10) ausgebildet ist zum Blockieren der Zuleitung der Hochfrequenzenergie zur Elektrodenanordnung in Reaktion auf das Signal.

11. Elektrochirurgisches System nach einem der vorhergehenden Ansprüche, **dadurch gekennzeichnet, dass** der Generator (10) oder die Speichereinrichtung (50) ausgebildet sind zum Speichern von Informationen über die Häufigkeit, mit der das Signal erzeugt wurde, wenn das elektrochirurgische Instrument (12) mit dem Generator verbunden wurde.

12. Elektrochirurgisches System nach Anspruch 1, **dadurch gekennzeichnet, dass** der Generator ausgebildet ist, unterschiedlich zu reagieren in Abhängigkeit davon, wie oft das Signal erzeugt wurde.

13. Verfahren zum Überwachen der erneuten Verwendung eines elektrochirurgischen Instruments (12), umfassend die Schritte:
i) das lösbare Verbinden des elektrochirurgischen Instruments (12) mit einem elektrochirurgischen Generator (10),
ii) das Detektieren, ob das elektrochirurgische Instrument vorher mit einem elektrochirurgischen Generator lösbar verbunden wurde,
iii) das Schreiben eines Werts von einem Aufwärtszähler (51), der in dem Generator untergebracht ist, in einen in dem elektrochirurgischen Instrument vorhandenen Speicher (50), wenn das elektrochirurgische Instrument vorher nicht mit einem elektrochirurgischen Generator lösbar verbunden wurde, und
iv) das Erhöhen des Werts des Aufwärtszählers um eins, in Reaktion auf das lösbare Verbinden des elektrochirurgischen Instruments.

14. Verfahren zum Überwachen der erneuten Verwendung nach Anspruch 13, ferner **gekennzeichnet durch** die Schritte:
v) das Vergleichen des vorliegenden Werts des Aufwärtszählers (51) mit dem Wert, der in dem in dem elektrochirurgischen Instrument (12) vorhandenen Speicher (50) gespeichert ist, wenn das elektrochirurgische Instrument (12) vorher mit einem elektrochirurgischen Generator (10) verbunden wurde, und
vi) das Generieren eines Signals, wenn die Differenz zwischen dem aktuellen Wert und dem gespeicherten Wert einen vorgegebenen Schwellwert übersteigt.

## Revendications

1. Système électrochirurgical comprenant :
un générateur (10) pour générer une puissance radio fréquence, et
un instrument électrochirurgical (12) incluant un ensemble électrode, l'instrument électrochirurgical pouvant être raccordé de manière détachable au générateur de telle sorte que la puissance radio fréquence peut être acheminée jusqu'à l'ensemble électrode,
**caractérisé en ce que** le générateur inclut un compteur (51) qui est adapté pour que sa valeur soit incrémentée chaque fois qu'un instrument électrochirurgical est raccordé de manière détachable au générateur, l'instrument électrochirurgical inclut un dispositif mémoire (50) adapté pour enregistrer la valeur du compteur lorsque l'instrument électrochirurgical est raccordé au générateur, et le système inclut une unité de comparaison (53) adaptée pour comparer la valeur actuelle du compteur à la valeur du compteur stockée dans le dispositif mémoire, et adaptée pour générer un signal lorsque la différence entre les deux valeurs du compteur est supérieure à un seuil prédéterminé.

2. Système électrochirurgical selon la revendication 1, **caractérisé en ce que** le seuil prédéterminé est supérieur à un.

3. Système électrochirurgical selon la revendication 2, **caractérisé en ce que** le seuil prédéterminé est compris entre 5 et 10.

4. Système électrochirurgical selon la revendication 2, **caractérisé en ce que** le seuil prédéterminé est supérieur à 10.

5. Système électrochirurgical selon l'une quelconque des revendications précédentes, **caractérisé en ce que** l'unité de comparaison (53) est présente dans le générateur (10).

6. Système électrochirurgical selon l'une quelconque des revendications 1 à 4, **caractérisé en ce que** l'unité de comparaison (53) est présente dans le système électrochirurgical (12).

7. Système électrochirurgical selon l'une quelconque des revendications précédentes, **caractérisé en ce que** le générateur (10) est adapté pour afficher un message d'avertissement en réponse au signal.

8. Système électrochirurgical selon l'une quelconque des revendications précédentes, **caractérisé en ce que** le générateur (10) est adapté pour générer une alarme audible en réponse au signal.

9. Système électrochirurgical selon l'une quelconque des revendications précédentes, **caractérisé en ce que** le générateur (10) est adapté pour réduire la puissance de la radio fréquence alimentée à l'ensemble électrode en réponse au signal.

10. Système électrochirurgical selon l'une quelconque des revendications précédentes, **caractérisé en ce que** le générateur (10) est adapté pour bloquer l'alimentation en puissance radio fréquence à l'ensemble électrode en réponse au signal.

11. Système électrochirurgical selon l'une quelconque des revendications précédentes, **caractérisé en ce que** le générateur (10) ou le dispositif mémoire (50) est adapté pour stocker des informations relatives au nombre de fois où le signal a été généré lorsque l'instrument électrochirurgical (12) a été raccordé au générateur.

12. Système électrochirurgical selon la revendication 11, **caractérisé en ce que** le générateur (10) est adapté pour faire une réponse différente en fonction du nombre de fois où le signal a été généré.

13. Procédé de surveillance de la réutilisation d'un instrument électrochirurgical (12) comprenant les étapes de :
i) raccord détachable de l'instrument électrochirurgical (12) à un générateur électrochirurgical (10),
ii) détection du fait que l'instrument électrochirurgical a été ou non préalablement raccordé de manière détachable à un générateur électrochirurgical,
iii) si l'instrument électrochirurgical n'a pas préalablement été raccordé de manière détachable à un générateur électrochirurgical, écriture d'une valeur d'un compteur incrémentiel (51), hébergé dans un générateur, dans une mémoire (50) présente dans l'instrument électrochirurgical, et
iv) incrémentation de la valeur du compteur incrémentiel en réponse au raccordement détachable de l'instrument électrochirurgical.

14. Procédé de surveillance de la réutilisation selon la revendication 13, **caractérisé par** les étapes supplémentaires de :
v) si l'instrument électrochirurgical (12) a préalablement été raccordé à un générateur électrochirurgical (10), comparaison de la valeur actuelle du compteur incrémentiel (51) à la valeur stockée dans la mémoire (50) présente dans l'instrument électrochirurgical (12), et
vi) génération d'un signal si la différence entre la valeur actuelle et la valeur stockée excède un seuil prédéterminé.
